# EUROPEAN PATENT APPLICATION

(11) **EP 2 433 568 A1**
(43) Date of publication of application: **28.03.2012**
(21) Application number: 11163078.6
(22) Date of filing: 19.04.2011
(51) Int. Cl.: A61B 8/08, G01S 15/89

(54) **Ultrasound system for combining images of various angles and method for operating ultrasound system**

(30) Priority: 24.09.2010 KR 20100092823
(71) Applicant: Samsung Medison Co., Ltd., Nam-myun Hongchun-gun Kangwon do 250-875 (KR)
(72) Inventor: Lee, Kwang-Hee, 302-743, Daejeon (KR); Kim, Jeong-Sik, 467-712, Gyeonggi-Do (KR)
(74) Representative: Schmid, Wolfgang

(57) **Abstract**

Provided are an ultrasound system and a method for operating the ultrasound system, which may irradiate an ultrasonic beam at various angles, may process a reflected ultrasonic echo signal to generate partial images, and may combine the partial images. The ultrasound system may include a beam irradiating unit to irradiate an ultrasonic beam onto an object at different irradiation angles for each frame, an image processing unit to process a reflected and returned ultrasonic echo signal to generate a partial image, and an image combining unit to combine and render the partial images generated at different irradiation angles for each frame to extend an angle of view of the object.

## Description

### CROSS-REFERENCE TO RELATED APPLICATION

This application claims the benefit of Korean Patent Application No. 10-2010-0092823, filed on September 24, 2010, in the Korean Intellectual Property Office, the disclosure of which is incorporated herein by reference.

### BACKGROUND

### 1. Field of the Invention

The present invention relates to an ultrasound system for combining images obtained by irradiating an ultrasonic beam at various irradiation angles and processing a reflected ultrasonic echo signal, and a method for operating the ultrasound system.

### 2. Description of the Related Art

An ultrasound system is an apparatus for transmitting, from the surface of the body, an ultrasound wave signal toward a predetermined structure, that is, an object such as a fetus or an internal organ, inside the body, and for visualizing a cross section of soft tissues or a blood flow using information of an ultrasound wave echo signal reflected from the tissues of the body.

This ultrasound system has advantages of a small size, a low cost, a real-time display, and a high stability without exposing patients and users to X-ray radiation, and thus, the ultrasound system is widely used along with other diagnostic imaging systems such as an X-ray diagnosis equipment, a computerized tomography (CT) scanner, a magnetic resonance imaging (MRI) equipment, a nuclear medicine diagnosis equipment, and the like.

The ultrasound system transmits an ultrasonic beam toward a predetermined region in the body, and obtains and outputs an image using an ultrasonic echo signal reflected from the tissues of the body. When an angle of the ultrasonic echo signal reflected from the tissues of the body is at a right angle, the obtained image is clearer. Currently, however, the ultrasound system does not apply a direction of the ultrasonic echo signal to obtain an image.

### SUMMARY

An aspect of the present invention provides an ultrasound system and a method for operating the ultrasound system, which may allow observation of an object at various angles by irradiating an ultrasonic beam onto the object at different irradiation angles for each frame.

Another aspect of the present invention provides an ultrasound system and a method for operating the ultrasound system, which may process an ultrasonic echo signal reflected from an object and returned to a probe, to generate a partial image of high resolution.

Still another aspect of the present invention provides an ultrasound system and a method for operating the ultrasound system, which may combine and render partial images generated at different irradiation angles of an ultrasonic beam, thereby extending an angle of view of an object.

According to an aspect of the present invention, there is provided an ultrasound system for combining images of various angles including a beam irradiating unit to irradiate an ultrasonic beam onto an object at different irradiation angles for each frame, an image processing unit to process a reflected and returned ultrasonic echo signal to generate a partial image, and an image combining unit to combine and render the partial images generated at different irradiation angles for each frame to extend an angle of view of the object.

According to another aspect of the present invention, there is provided a method for operating an ultrasound system for combining images of various angles including irradiating an ultrasonic beam onto an object at different irradiation angles for each frame, processing a reflected and returned ultrasonic echo signal to generate a partial image, and combining and rendering the partial images generated at different irradiation angles for each frame to extend an angle of view of the object.

### EFFECT OF THE INVENTION

According to an aspect of the present invention, provided are an ultrasound system and a method for operating the ultrasound system, which may allow observation of the object at various angles by irradiating an ultrasonic beam onto the object at different irradiation angles for each frame, thereby extending an angle of view of the object.

According to another aspect of the present invention, provided are an ultrasound system and a method for operating the ultrasound system, which may process an ultrasonic echo signal of an irradiated ultrasonic beam reflected from an object and returned to a probe, to generate a partial image of high resolution, thereby obtaining a high-resolution image by processing the ultrasonic echo signal.

According to still another aspect of the present invention, provided are an ultrasound system and a method for operating the ultrasound system, which may combine and render partial images generated at different irradiation angles of an ultrasonic beam to generate an ultrasonic image including an object, thereby extending an angle of view of the object for observing the object at various angles.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a view illustrating an example of combining partial images generated by irradiating an ultrasonic beam at different irradiation angles according to an embodiment of the present invention;
FIG. 2 is a block diagram illustrating an internal structure of an ultrasound system according to an embodiment of the present invention; and
FIG. 3 is a flowchart illustrating a method for operating the ultrasound system according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

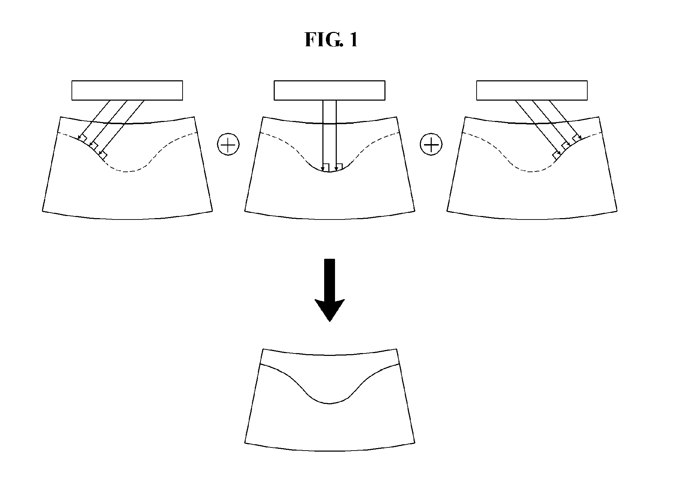

FIG. 1 is a view illustrating an example of combining partial images generated by irradiating an ultrasonic beam at different irradiation angles according to an embodiment of the present invention.

The ultrasound system may irradiate an ultrasonic beam onto an object at different irradiation angles by controlling a probe. The ultrasound system may irradiate an ultrasonic beam onto the object in different output directions of the ultrasonic beam, that is, at different irradiation angles.

The ultrasound system may receive an ultrasonic echo signal reflected from the object at a right angle for each of the irradiation angles, and may perform signal processing of the received ultrasonic echo signal to generate a partial image. The ultrasound system may receive the ultrasonic echo signal corresponding to the irradiation angle, reflected from the object at a right angle, and may process the ultrasonic beam as a signal, to generate a partial image for the irradiation angle.

The ultrasound system may combine and render the partial images corresponding to each of the irradiation angles to generate an image of the object, and may display the image. The ultrasound system may combine and render the partial images generated for each of the irradiation angles to generate an image of the object with a wide angle of view, and may display the image.

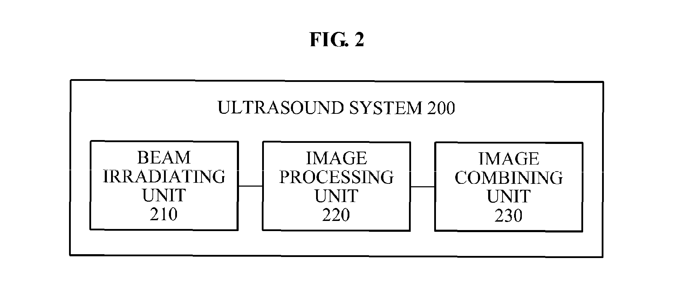

FIG. 2 is a block diagram illustrating an internal structure of an ultrasound system 200 according to an embodiment of the present invention.

Referring to FIG. 2, the ultrasound system 200 for combining images of different irradiation angles according to an embodiment of the present invention may include a beam irradiating unit 210, an image processing unit 220, and an image combining unit 230.

The beam irradiating unit 210 may irradiate an ultrasonic beam onto an object in different output directions of the ultrasonic beam, that is, at different irradiation angles, while a probe is fixed. The beam irradiating unit 210 may irradiate an ultrasonic beam onto an object at an irradiation angle varying within a predetermined angle range. The beam irradiating unit 210 may irradiate an ultrasonic beam onto an object at various irradiation angles, may sense an ultrasonic echo signal reflected from the object, that is, sensing data, and may transmit the sensing data to the image processing unit 220.

The image processing unit 220 may process the sensing data received from the beam irradiating unit 210, that is, the reflected and returned ultrasonic echo signal, to generate a partial image. The image processing unit 220 may process the ultrasonic echo signal outputted at a predetermined irradiation angle and reflected from the object at a right angle, to generate a partial image. The image processing unit 220 may transmit the generated partial image to the image combining unit 230.

The image combining unit 230 may combine and render the partial images generated at different irradiation angles to generate an image of the object for observing the object at various angles. The image combining unit 230 may combine and render the partial images generated at each of the irradiation angles to generate one image including the object.

When the object is the nuchal translucency (NT), the beam irradiating unit 210, the image processing unit 220, and the image combining unit 230 of the ultrasound system 200 may operate as described below.

The beam irradiating unit 210 may irradiate an ultrasonic beam onto the NT at different irradiation angles while a probe is fixed. The beam irradiating unit 210 may irradiate an ultrasonic beam around the NT in different output directions of the ultrasonic beam, that is, at different irradiation angles, while the probe is located near the NT.

The image processing unit 220 may process the ultrasonic echo signal of the ultrasonic beam irradiated onto the NT and reflected and returned from the NT at a right angle to generate a partial image. The image processing unit 220 may perform signal processing of the ultrasonic echo signal of the ultrasonic beam irradiated onto the NT at different irradiation angles and reflected from the NT at a right angle, to generate a partial image.

The image combining unit 230 may combine and render the partial images generated at different irradiation angles to generate an image of the NT for observing the NT at various angles. The image combining unit 230 may combine and render the partial images of different irradiation angles, thereby extending an angle of view of the NT.

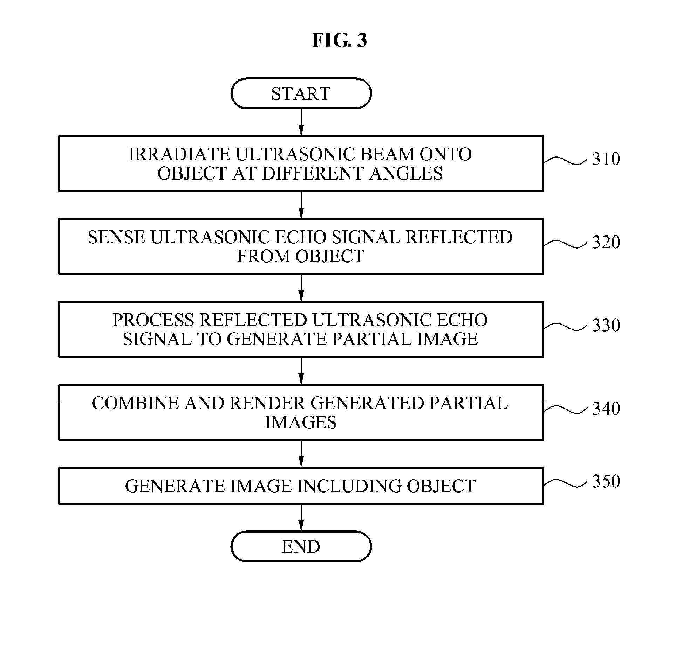

FIG. 3 is a flowchart illustrating a method for operating the ultrasound system according to an embodiment of the present invention.

The method for operating the ultrasound system according to an embodiment of the present invention may be implemented by the ultrasound system of FIG. 2. Hereinafter, the description of FIG. 3 is made with reference to FIG. 2 for ease of description.

In operation 310, the ultrasound system may irradiate an ultrasonic beam onto an object in different output directions of the ultrasonic beam, that is, at different irradiation angles, while a probe is fixed. The ultrasound system may irradiate an ultrasonic beam onto an object at an irradiation angle varying within a predetermined angle range.

In operation 320, the ultrasound system may sense an ultrasonic echo signal of the ultrasonic beam irradiated onto the object at different irradiation angles and reflected from the object to obtain sensing data.

In operation 330, the ultrasound system may process the sensing data, that is, the reflected and returned ultrasonic echo signal to generate a partial image. The ultrasound system may process the ultrasonic echo signal outputted at a predetermined irradiation angle and reflected from the object at a right angle, to generate a partial image.

In operation 340, the ultrasound system may combine and render the partial images generated at different irradiation angles.

In operation 350, the ultrasound system may generate an image of the object for observing the object at various angles. The ultrasound system may generate one image including the object.

The above-described exemplary embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. An ultrasound system for combining images of various angles, the system comprising:
a beam irradiating unit to irradiate an ultrasonic beam onto an object at different irradiation angles for each frame;
an image processing unit to process a reflected and returned ultrasonic echo signal to generate a partial image; and
an image combining unit to combine and render the partial images generated at different irradiation angles for each frame to extend an angle of view of the object.

2. The system of claim 1, wherein the beam irradiating unit irradiates the ultrasonic beam onto the object at different irradiation angles for each frame while a probe is fixed.

3. The system of claim 1, wherein when the image processing unit processes the ultrasonic echo signal reflected and returned at a right angle, to generate a partial image.

4. The system of claim 1, wherein the image combining unit combines and renders the partial images generated for each frame to generate one image including the object.

5. The system of claim 1, wherein when the object is the nuchal translucency (NT), the beam irradiating unit irradiates the ultrasonic beam onto the NT at different irradiation angles while a probe is fixed,
the image processing unit processes the ultrasonic echo signal of the ultrasonic beam irradiated onto the NT and reflected and returned at a right angle, to generate a partial image, and
the image combining unit combines and renders the partial images generated at different irradiation angles for each frame to extend an angle of view of the NT.

6. A method for operating an ultrasound system for combining images of various angles, the method comprising:
irradiating an ultrasonic beam onto an object at different irradiation angles for each frame;
processing a reflected and returned ultrasonic echo signal to generate a partial image; and
combining and rendering the partial images generated at different irradiation angles for each frame to extend an angle of view of the object.

7. The method of claim 6, wherein the irradiating of the ultrasonic beam onto the object comprises irradiating the ultrasonic beam onto the object at different irradiation angles for each frame while a probe is fixed.

8. The method of claim 6, wherein the generating of the partial image comprises generating the partial image by processing the ultrasonic echo signal reflected and returned at a right angle.

9. The method of claim 6, wherein the combining and rendering of the partial images comprises combining and rendering the partial images generated for each frame to generate one image including the object.
